(19) Europäisches Patentamt

European Patent Office

Office européen des brevets

(11) Veröffentlichungsnummer: **0 506 900 B1**

(12) **EUROPÄISCHE PATENTSCHRIFT**

(45) Veröffentlichungstag der Patentschrift: **09.08.95**

(21) Anmeldenummer: **91917358.3**

(22) Anmeldetag: **16.10.91**

(86) Internationale Anmeldenummer:
**PCT/CH91/00215**

(87) Internationale Veröffentlichungsnummer:
**WO 92/06698 (30.04.92 92/10)**

(51) Int. Cl.⁶: **A61K 35/78**

(54) **VERFAHREN ZUR HERSTELLUNG VON SPAGYRISCHEN ESSENZEN AUS PFLANZEN.**

(30) Priorität: **22.10.90 CH 3353/90**

(43) Veröffentlichungstag der Anmeldung:
**07.10.92 Patentblatt 92/41**

(45) Bekanntmachung des Hinweises auf die
Patenterteilung:
**09.08.95 Patentblatt 95/32**

(84) Benannte Vertragsstaaten:
**AT BE CH DE DK ES FR GB GR IT LI LU NL SE**

(56) Entgegenhaltungen:
**DE-A- 1 617 314**
**FR-A- 1 539 878**

**Patent Abstracts of Japan, vol.9, No.228 (C-303)(1951) 13 September 1985 & JP-A-60 087 207**

(73) Patentinhaber: **KRUMMENACHER, Beat Rudolf**
**Tägertschistrasse 34a**
**CH-3110 Münsingen (CH)**

(72) Erfinder: **KRUMMENACHER, Beat Rudolf**
**Tägertschistrasse 34a**
**CH-3110 Münsingen (CH)**

(74) Vertreter: **Feldmann, Clarence Paul et al**
**c/o Patentanwaltsbüro FELDMANN AG**
**Postfach**
**Kanalstrasse 17**
**CH-8152 Glattbrugg (CH)**

EP 0 506 900 B1

## Beschreibung

Die vorliegende Erfindung betrifft ein Verfahren zur Herstellung von spagyrischen Essenzen aus frischen oder getrockneten Pflanzen oder Pflanzenteilen.

Es sind verschiedene Verfahren bekannt, die zum Ziel haben, Arzneimittel auf pflanzlicher Basis so herzustellen, dass die heilkräftigen Prinzipien der Pflanzen erhalten werden.

So zeigt DE-A-1'617'314 (Bika GmbH) ein Verfahren zur Gewinnung von prophylaktisch wirkenden Weinen aus Heilpflanzen. Die Pflanzen werden unter Zugabe von Wasser, Glucose und Hefe vergärt. Durch Sieben und Pressen wird anschliessend die fermentierte Flüssigkeit von den festen Bestandteilen getrennt. Nach der Beigabe von Honig wird nochmals vergärt, anschliessend wird zur Konservierung Benzoesäure hinzugefügt. Nach der Filtration wird ein Honigtrinkwein erhalten.

FR-A-1'539'878 (Savard) zeigt ein Verfahren zur Herstellung von Urtinkturen aus pflanzlicher Basis, bei dem die Pflanzen zerkleinert und anschliessend in destilliertem Wasser vergoren werden. Anschliessend wird durch Filtration und Pressen der Pflanzenrückstand von der Flüssigkeit getrennt. Der Pflanzenrückstand wird verascht und die Asche in die Flüssigkeit gegeben, so dass sich die löslichen Mineralsalze darin auflösen können. Nach erneuter Filtration wird die erhaltene Lösung dem Lichte ausgesetzt, bis eine klare Flüssigkeit erhalten wird.

Jp-A-60'87207 (Iwasekenjirou Shiyouten K.K.) zeigt ein Verfahren zur Gewinnung eines vergorenen Auszuges aus Aloearten (Aloe genus, Liliaceae family) zur äusserlichen Anwendung.

Alle Verfahren weisen am Anfang eine Vergärung auf, auf die einfache Trennprozesse folgen.

Im spagyrischen Verfahren nach Zimpel werden frische Pflanzen zerkleinert und unter Zugabe von Wasser und Hefe einer Gärung ausgesetzt. Wenn der Gärvorgang beendet ist, wird die vergorene Masse einer Destillation unterworfen. Dem Destillat wird Aethanol zugegeben. Der Destillationsrückstand wird abgepresst, getrocknet und verascht. Der Veraschungsrückstand wird anschliessend zum Destillat gegeben und filtriert.

Es ist daher Aufgabe der vorliegenden Erfindung, ein Verfahren zur Herstellung von spagyrischen Essenzen auf pflanzlicher Basis aufzuzeigen, das zu einer vordefinierten Produktmenge mit vorausbestimmtem Alkoholgehalt führt, also standardisiert ist.

Diese Aufgabe löst ein Verfahren mit den Merkmalen des Patentanspruchs 1. Weitere vorteilhafte Ausgestaltungsformen gehen aus den abhängigen Patentansprüchen hervor.

Im erfindungsgemässen Verfahren zur Herstellung von spagyrischen Essenzen bzw. Urtinkturen aus frischen oder getrockneten Pflanzen bzw. Pflanzenteilen, werden alle Herstellungszusätze, Zwischenproduktmengen sowie die Endproduktmenge standardisiert bezüglich der Trockenmasse der verwendeten Pflanze. Das Endprodukt ( = spagyrische Essenz bzw. Urtinktur) besitzt einen definierten Alkoholgehalt und läuft wie folgt ab:

Saccharose, Honig oder andere, vergärbare Kohlehydrate werden in Trinkwasser gelöst und Hefe eingerührt. Die fein zerkleinerte Pflanzenmasse wird hinzugefügt und alles gründlich durchmischt. Die Pflanzenmasse soll dabei mindestens knapp von Flüssigkeit bedeckt sein. Dann wird unter täglich mindestens einmaligem, gründlichem Durchmischen bei einer geeigneten Temperatur vergoren. Sind die Gärvorgänge zum Stillstand gekommen, wird die gesamte Masse unter Vakuum destilliert. Man sammelt zwei Fraktionen, wobei die erste Fraktion sämtlichen aus der Gärung stammenden Alkohol enthalten muss und derart bemessen wird, dass sie zusammen mit dem zum Erreichen des Sollalkoholgehalts im Endprodukt erforderlichen Alkoholzusatz (Aethanolum 94%) sowie den zuzusetzenden Salzen die Endproduktmenge nicht überschreitet. Bei der Bestimmung des erforderlichen Alkoholzusatzes ist die in Fraktion 1 enthaltene Alkoholmenge mitzuberücksichtigen.

Der gesamte Destillationsrückstand wird getrocknet, verbrannt und bei einer geeigneten Temperatur vollständig verascht. Die Asche darf dabei weder verschlacken noch schmelzen. Aus der Asche werden mit einem spezifischen Lösungsmittel sämtliche darin löslichen Mineralsalze erschöpfend extrahiert, mitextrahierte Verunreinigungen entfernt, die gereinigten Salzextrakte vereint und zur Trockene gebracht. Die getrockneten Salze werden in einem zur Auflösung genügenden Anteil der mit der zur Gehaltseinstellung erforderlichen Alkoholmenge vermischten Fraktion 1 aufgelöst. Ein allfällig sich bildender Niederschlag wird abfiltriert. Man vermischt mit dem Rest von Fraktion 1 und fügt - falls erforderlich - soviel von Fraktion 2 hinzu, bis die Gesamtmasse mit der Endproduktmenge übereinstimmt. Die resultierende, klare Flüssigkeit ist die spagyrische Essenz bzw. Urtinktur aus der verwendeten Pflanze.

Zur Extraktion der Salze aus der Asche wird als spezifisches Lösungsmittel bevorzugterweise gereinigtes Wasser (Aqua purificata) verwendet. Die Entfernung mitextrahierter Verunreinigungen besteht in der alleinigen Abfiltration eines sich allfällig abscheidenden Niederschlags nach Auflösung der vereinigten und zur Trockene gebrachten Salzextrakte in einem zur Auflösung genügenden Anteil der mit der zur Gehaltseinstellung erforderlichen Alkoholmenge vermischten Fraktion 1. In einer erweiterten Form des Verfahrens erfolgt die Entfernung mitextrahierter Verunreinigungen dadurch, dass die vereinigten und zur Trockene gebrachten Salzextrakte in weiterem gereinigtem Wasser unter Kochen gelöst und ein sich allfällig abscheidender Niederschlag abfiltriert wird. Man bringt zur Trockene und löst die Salze in einem zur Auflösung genügenden Anteil der mit der zur Gehaltseinstellung erforderlichen Alkoholmenge vermischten Fraktion 1.

Dieser Reinigungsprozess der vereinigten und zur Trockene gebrachten Salzextrakte durch Auflösen in gereinigtem Wasser unter Kochen und Abfiltration eines sich allfällig abscheidenden Niederschlags kann sooft wiederholt werden, bis sich nichts mehr abscheidet, das heisst sich die gesamten Salze nach erneutem Aufkochen in gereinigtem Wasser klar lösen. Erst dann bringt man schliesslich zur Trockene und löst die Salze in einem zur Auflösung genügenden Anteil der mit der zur Gehaltseinstellung erforderlichen Alkoholmenge vermischten Fraktion 1.

In einer weiteren Variante werden die vereinigten und zur Trockene gebrachten Salzextrakte beziehungsweise getrockneten Salze vor Auflösen in gereinigtem Wasser unter Kochen jeweils bei einer geeigneten Temperatur geglüht, wobei sie weder verschlacken noch schmelzen dürfen.

In einer weiteren Variante wird das gereinigte Wasser durch einen Teil von Fraktion 2 ersetzt.

In einer weiteren Variante wird zur erschöpfenden Extraktion der Salze aus der Asche als spezifisches Lösungsmittel destillierter Weinessig oder ein anderer, destillierter und mittels Essigbakterien aus alkoholischen Flüssigkeiten gewonnener Essig im Ueberschuss verwendet. Die sauren Salzextrakte werden vereint und unter Vakuum zur Trockene gebracht. Zur Entfernung letzter Reste überschüssiger Essigsäure werden die getrockneten Salze in gereinigtem Wasser gelöst und erneut unter Vakuum zur Trockene gebracht. Dieses Auflösen der Salze in gereinigtem Wasser und erneute Abdestillieren des Wassers unter Vakuum wird sooft wiederholt, bis das Destillat nicht mehr sauer reagiert. Die Entfernung mitextrahierter Verunreinigungen erfolgt durch Abfiltration eines sich allfällig abscheidenden Niederschlags nach Auflösung der getrockneten und von überschüssiger Säure befreiten Salze in einem zur Auflösung genügenden Anteil der mit der zur Gehaltseinstellung erforderlichen Alkoholmenge vermischten Fraktion 1.

Die Entfernung mitextrahierter Verunreinigungen kann jedoch auch dadurch erfolgen, dass die getrockneten Salze in gereinigtem Wasser unter Kochen gelöst und ein sich allfällig abscheidender Niederschlag abfiltriert werden.

Dieser Vorgang kann sooft wiederholt werden, bis sich nichts mehr abscheidet, dass heisst sich die gesamten Salze unter Kochen in gereinigtem Wasser klar lösen.

Die Bestandteile für den Gäransatz sind in der angegebenen Reihenfolge zu verarbeiten. Insbesondere darf das Pflanzenmaterial erst am Schluss zur kohlehydrathaltigen Hefelösung hinzugefügt werden. Nur dies gestattet eine steuerbare Hefegärung, indem die Hefeentwicklung gefördert und das Wachstum unerwünschter Keime verhindert wird. Einzelne Pflanzenarten enthalten kaum vergärbare Kohlehydrate. Um die für die Herstellung spagyrischer Essenzen wichtigen Gärprozesse in jedem Fall kräftig in Gang zu setzen, wird ein Kohlehydratzusatz verlangt. Die Gärvorgänge sollen möglichst optimal ablaufen. Die Gärtemperatur ist daher in Abhängigkeit der verwendeten Hefe festzulegen. Erst nach vollständig abgeschlossener Vergärung darf der Ansatz weiterverarbeitet werden. Dieser Zeitpunkt ist dann erreicht, wenn keine Gärgase (Kohledioxid u.a.) mehr gebildet werden und in der Regel die Pflanzenteile auf den Gefässgrund abgesunken sind. Um die für spagyrische Essenzen wichtigen, oft temperaturempfindlichen Aromastoffe möglichst vollständig und unzersetzt zu erhalten, wird die besonders schonende Vakuumdestillation vorgeschrieben. Weil die Endproduktmenge in einem festen Verhältnis zur Trockenmasse der verwendeten Ausgangspflanze steht, muss das Destillat in zwei Fraktionen gesammelt werden. Die für die Qualität einer spagyrischen Essenz wesentlichen Aromastoffe sowie der durch die Gärprozesse entstandene Alkohol destillieren vorwiegend zu Beginn, während ihr Anteil am Destillat mit fortlaufender Destillation immer mehr abnimmt. Daher soll Fraktion 1 möglichst gross sein, darf jedoch zusammen mit den späteren Zusätzen die zu erreichende Endproduktmenge nicht überschreiten. Die praktisch nur noch aus Wasser bestehende Fraktion 2 dient der exakten Einstellung der Endproduktmenge im Sinne der Standardisierung sowie bei Patentanspruch 6 zusätzlich der Gewinnung der löslichen Mineralsalze.

Der Alkoholgehalt ist so festzulegen, dass folgende Bedingungen erfüllt werden:

- Alle im Destillat erscheinenden, mehr oder weniger hydrophoben Bestandteile (insbesondere ätherische Oele) sollen im Produkt in klarer Lösung vorliegen. Diese Forderung wird durch Erhöhung des Alkoholgehaltes erfüllt.

- Sämtliche aus der Asche gewonnenen Mineralsalze sollen im Endprodukt ebenfalls in klarer Lösung vorliegen. Diese Forderung wird durch Erniedrigung des Alkoholgehaltes erfüllt.

- Eine sichere Konservierung muss gewährleistet sein. Diese Forderung liefert den Minimalwert für den Alkoholgehalt.

Zur Alkoholgehaltseinstellung wird der Alkoholgehalt von Fraktion 1 bestimmt. Dann errechnet man unter Einbezug der in Fraktion 1 vorhandenen Alkoholmenge die noch zuzusetzende Menge Aethanol 94 %, um in der Endproduktmenge den festgelegten Alkoholgehalt zu erreichen.

Vollständige Veraschung bedeutet Oxidation sämtlicher organischer Bestandteile, zurück bleiben nur die anorganischen Mineralstoffe. Die Veraschung muss also oxidativ erfolgen. Die Temperatur ist dabei so zu wählen, dass zwar vollständige Veraschung eintritt, die Asche aber weder verschlackt noch zusammenschmilzt.

Zur erschöpfenden Extraktion der Mineralsalze aus der Asche wird ein geeignetes Verfahren angewendet. In Frage kommen wiederholte Mazeration oder Digestion, Soxhletextraktion u.a. Bei den Patentansprüchen 2 bis 6 wird zur erschöpfenden Extraktion gereinigtes Wasser (Aqua purificata) verwendet. Dieses kann durch einen Teil von Fraktion 2 (Patentanspruch 7) oder mittels biologischer Verfahren gewonnenen, destillierten Essig (Patentansprüche 8 bis 10) ersetzt werden. Je nach dem verwendeten Extraktionsmittel kommen unterschiedliche Reinigungsverfahren der isolierten Salze in Frage. Sie ergeben sich aus der Tatsache, dass aus spagyrischer Sicht Bestandteile der extrahierten Salze als Verunreinigungen anzusehen sind, wenn sie bei den in den Patentansprüchen 2 bis 10 beschriebenen verschiedenen Ausführungsformen als Niederschläge anfallen.

Spagyrische Essenzen beziehungsweise Urtinkturen werden als Arzneimittel in der Human- und Veterinärmedizin eingesetzt.

## Ausführungsbeispiel

Das Verhältnis zwischen der Trockenmasse der verwendeten Pflanzen und der Endproduktmenge beträgt 1:10. Der Alkoholgehalt wird auf 22,0% G/G eingestellt. Als Kohlehydrat wird Saccharose verwendet.

**Vorschrift:** Die Pflanzen oder Pflanzenteile werden fein zerkleinert. Von einer Probe wird der Trockenrückstand T in Prozent bestimmt. Mit der Gesamtpflanzenmasse M in Kilogramm berechnet man die Sollmenge Urtinktur U gemäss:

$$U = 0{,}1 \cdot M \cdot T \ [kg]$$

Die für den Gäransatz erforderlichen Mengen Wasser, Saccharose und Hefe werden nach den Formeln bestimmt:

$$\text{Wassermenge } W = U \ [kg]$$
$$\text{Saccharose } S = 0.03 \cdot U \ kg]$$
$$\text{Hefe } H = 0.002 \cdot U \ [kg]$$

Die gesamte Wassermenge wird in ein geeignetes Ansatzgefäss gegeben und darin die Saccharose aufgelöst. Dann wird die Hefe eingerührt, worauf die zerkleinerte Pflanzenmasse hinzugemischt wird. Sollte die Pflanzenmasse nicht mindestens knapp mit Flüssigkeit bedeckt sein, ist zusätzlich Wasser hinzuzufügen. Das Ansatzgefäss wird bei einer Temperatur von 15 bis 25 °C unter täglichem, gründlichem Umrühren und Hinunterstossen aufgestiegener Festbestandteile der Gärung überlassen. Sobald die Gärvorgänge zum Stillstand gekommen sind, wird die gesamte Masse unter Vakuum destilliert. Bei einem Druck von 135 mbar werden zwei Fraktionen gesammelt:

$$\text{Fraktion 1} = F1 = 0{,}7 \cdot U \ [kg]$$
$$\text{Fraktion 2} = F2 = 0{,}15 \cdot U \ [kg]$$

In der effektiv gesammelten Menge D der Fraktion 1 wird der Aethanolgehalt A (Gewichtsprozent) bestimmt. Dann fügt man Aethanol 94% (G/G)) in der Menge Z hinzu:

$$Z = (1/94) \cdot (22 \cdot U\text{-}A \cdot D) \ [kg]$$

Der Destillationsrückstand wird getrocknet, verbrannt und bei einer Temperatur von 480 - 520 °C vollständig verascht. Mit kochendem Wasser werden die löslichen Salze aus der Asche erschöpfend extrahiert. Man filtriert die Salzextrakte und bringt zur Trockene.

In einem zur Lösung der Salze genügenden Anteil der mit Alkohol vermischten Fraktion 1 werden die Salze gelöst. Dabei können sich Verunreinigungen absetzen, die abfiltriert werden. Die klare Lösung wird mit dem restlichen Anteil von alkoholischer Fraktion 1 vermischt. Von Fraktion 2 wird schliesslich soviel der Mischung hinzugefügt, bis die Gesamtmasse mit der Sollmenge U übereinstimmt. Die resultierende, klare Flüssigkeit ist die spagyrische Essenz beziehungsweise Urtinktur aus der verwendeten Pflanze.

## Patentansprüche

1. Verfahren zur Herstellung von spagyrischen Essenzen oder Urtinkturen aus frischen oder getrockneten Pflanzen oder Pflanzenteilen, wobei

   a) vergärbare Kohlenhydrate in Trinkwasser gelöst werden, Hefe eingerührt und die zerkleinerte Pflanzenmasse hinzugefügt wird,

   b) nach erstmaliger Durchmischung, wobei die Pflanzenmasse mindestens knapp von Flüssigkeit bedeckt ist, unter täglich mindestens einmaliger Durchmischung bei einer der verwendeten Hefe angepassten Temperatur vollständig vergoren wird,

c) anschliessend die gesamte, nach dem Schritt b) erhaltene Masse destilliert wird,

d) dem Destillat Alkohol zugegeben wird,

e) der gesamte Destillationsrückstand getrocknet, verbrannt und bei einer geeigneten Temperatur vollständig verascht wird, bevor er dem Destillat zugegeben wird,

dadurch gekennzeichnet

f) dass vor dem Schritt a) die Trockenmasse (M•T) der verwendeten Pflanze bestimmt und anhand dieser die Endproduktmenge (U), die Zwischenproduktmenge (F1) einer ersten Fraktion einer Destillation und eine beizufügende Kohlenhydratmenge sowie Hefemenge vordefiniert werden,

g) dass unter Vakuum destilliert wird, wobei zwei Fraktionen gesammelt werden, von denen die erste sämtlichen aus der Gärung stammenden Alkohol enthält und die zweite Fraktion aus dem nach Sammeln der ersten Fraktion erhaltenen Anteil des Destillats besteht,

h) dass der Alkoholgehalt der ersten Fraktion bestimmt wird und ihr Alkohol zugegeben wird, bis zur Erreichung eines Sollalkoholgehaltes bezogen auf die Endproduktmenge (U),

i) dass der Destillationsrückstand unter Vermeidung von Verschlackung oder Schmelzung verascht wird, und dass aus der Asche mit einem spezifischen Lösungsmittel sämtliche darin löslichen Mineralsalze erschöpfend extrahiert und anschliessend gereinigt werden: worauf die gereinigten Salzextrakte wieder vereint und zur Trockene gebracht werden,

k) die getrockneten Salze in einem zur Auflösung genügenden Teil der mit Alkohol vermischten ersten Fraktion aufgelöst werden und ein allfällig sich bildender Niederschlag abfiltriert wird,

l) die Lösung mit dem Rest der alkoholischen ersten Fraktion vermischt wird und

m) zum Schluss soviel von der zweiten Fraktion hinzugefügt wird, bis die Gesamtmasse mit der vordefinierten Endproduktmenge übereinstimmt.

2. Verfahren nach Anspruch 1, dadurch gekennzeichnet, dass als vergärbare Kohlenhydrate Saccharose oder Honig verwendet wird.

3. Verfahren nach Anspruch 1, dadurch gekennzeichnet, dass als spezifisches Lösungsmittel gereinigtes Wasser verwendet wird.

4. Verfahren nach Anspruch 3, dadurch gekennzeichnet, dass die vereinigten und zur Trockene gebrachten Salzextrakte in zusätzlichem, gereinigtem Wasser unter Kochen erneut gelöst werden, ein sich allfällig bildender Niederschlag abfiltriert und die Lösung zur Trockene gebracht wird.

5. Verfahren nach Anspruch 4, dadurch gekennzeichnet, dass die Salzextrakte erneut in gereinigtem Wasser unter Kochen gelöst werden, ein sich allfällig bildender Niederschlag abfiltriert und die Lösung zur Trockene gebracht wird, und dass diese Vorgänge solange wiederholt werden, bis sich die gesamten Salze beim Aufkochen in gereinigtem Wasser klar lösen, und dass diese Lösung abschliessend zur Trockene gebracht wird.

6. Verfahren nach einem der Ansprüche 3 bis 5, dadurch gekennzeichnet, dass die vereinigten und zur Trockene gebrachten Salzextrakte jeweils vor Auflösen in gereinigtem Wasser geglüht werden, ohne zu verschlacken oder zu schmelzen.

7. Verfahren nach einem der Ansprüche 3 bis 6, dadurch gekennzeichnet, dass das zur Entfernung mitextrahierter Verunreinigungen zur Salzextraktion verwendete, gereinigte Wasser durch einen Teil der zweiten Fraktion ersetzt wird.

8. Verfahren nach Anspruch 1, dadurch gekennzeichnet, dass

a) als Lösungsmittel destillierter Weinessig oder ein anderer, destillierter und mittels Essigbakterien aus alkoholischen Flüssigkeiten gewonnener Essig im Ueberschuss verwendet wird,

b) die sauren Salzextrakte vereint,

c) unter Vakuum zur Trockene gebracht,

d) die getrockneten Salze zur Entfernung letzter Reste überschüssiger Essigsäure in gereinigtem Wasser gelöst und

e) erneut unter Vakuum zur Trockene gebracht werden,

f) die Schritte d und e so oft wiederholt werden, bis das Destillat annähernd neutral reagiert.

9. Verfahren nach Anspruch 8, dadurch gekennzeichnet, dass die getrockneten und von aller überschüssigen Säure befreiten Salze zur Entfernung mitextrahierter Verunreinigungen in gereinigtem Wasser unter Kochen gelöst werden, ein sich allfällig bildender Niederschlag abfiltriert und die Lösung zur Trockene gebracht wird.

**10.** Verfahren nach Anspruch 9, dadurch gekennzeichnet, dass die Salze erneut in gereinigtem Wasser unter Kochen gelöst werden, ein sich allfällig bildender Niederschlag abfiltriert und die Lösung zur Trockene gebracht wird, und dass diese Vorgänge solange wiederholt werden, bis sich die gesamten Salze beim Aufkochen in gereinigtem Wasser klar lösen, und dass diese Lösung abschliessend zur Trockene gebracht wird.

**Claims**

**1.** Method for producing spagyric essences or primary tinctures from fresh or dried plants or plant parts, in which

a) fermentable carbohydrates are dissolved in drinking water, yeast is stirred in and the comminuted plant material is added,

b) following the first thorough mixing, in which the plant material is at least just covered with liquid, complete fermentation takes place at a temperature adapted to the yeast used and accompanied by thorough mixing at least once daily,

c) subsequently all the material obtained according to stage b) is distilled,

d) alcohol is added to the distillate,

e) all the distillation residue is dried, burnt and completely incinerated at a suitable temperature before being added to the distillate,

characterized in that

f) prior to stage a) the dry mass (M•T) of the plants used is determined and on the basis of this the end product quantity (U), the intermediate product quantity (F1) of a first fraction of a distillation and a carbohydrate quantity to be added, as well as the yeast quantity are previously defined,

g) that distillation takes place in vacuo, two fractions being collected, whereof the first contains all the alcohol resulting from the fermentation and the second fraction consists of the distillate portion obtained after collecting the first fraction,

h) that the alcohol content of the first fraction is determined and alcohol is added to it until a desired alcohol content, relative to the end product quantity (U) is obtained,

i) that the distillation residue is incinerated, whilst avoiding slagging or melting, and that from the ash and using a specific solvent all the mineral salts soluble therein are exhaustively extracted and subsequently purified, the purified salt extracts then being recombined and dried,

k) the dried salts are dissolved in a part of the first fraction mixed with alcohol adequate for dissolving purposes and any precipitate formed is filtered off,

l) the solution is mixed with the remainder of the alcoholic, first fraction and

m) finally sufficient of the second fraction is added until the total amount coincides with the previously defined end product quantity.

**2.** Method according to claim 1, characterized in that saccharose or honey is used as fermentable carbohydrates.

**3.** Method according to claim 1, characterized in that purified water is used as the specific solvent.

**4.** Method according to claim 3, characterized in that the combined, dried salt extracts are again dissolved, accompanied by boiling, in additional, purified water, any precipitate forming is filtered off and the solution is dried.

**5.** Method according to claim 4, characterized in that the salt extracts are again dissolved in purified water, accompanied by boiling, any precipitate formed is filtered off and the solution dried and that these processes are repeated until all the salts dissolve clearly when boiled in purified water and that this solution is subsequently dried.

**6.** Method according to one of the claims 3 to 5, characterized in that the combined and dried salt extracts are calcined, without slagging or melting prior to dissolving in purified water.

**7.** Method according to one of the claims 3 to 6, characterized in that for removing coextracted impurities purified water used for salt extraction is replaced by part of the second fraction.

**8.** Method according to claim 1, characterized in that as the solvent use is made in excess of distilled wine vinegar or some other, distilled vinegar obtained by means of vinegar bacteria from alcoholic liquids,

b) the acid salt extracts are combined,

c) are dried in vacuo,

d) the dried salts are dissolved in purified water for removing the final residues of excess acetic acid and

e) are again dried in vacuo,

f) stages d) and e) being repeated until the distillate reacts approximately neutral.

**9.** Method according to claim 8, characterized in that the dried salts freed from all excess acid are dissolved, accompanied by boiling, in purified water for removing coextracted impurities, any precipitate formed is filtered off and the solution is dried.

**10.** Method according to claim 9, characterized in that the salts are again dissolved in purified water, accompanied by boiling, any precipitate formed is filtered off and the solution dried and that these processes are repeated until all the salts dissolve clearly on boiling in purified water and that subsequently said solution is dried.

**Revendications**

**1.** Procédé pour la préparation d'essences ou de teintures originales spagyriques à partir de plantes ou de parties de plantes fraîches ou séchées, dans lequel

a) des hydrates de carbone fermentescibles sont dissous dans de l'eau potable, de la levure est introduite sous agitation et la masse végétale broyée y est ajoutée,

b) après un premier mélange intime, dans lequel la masse végétale est au moins tout juste recouverte par le liquide, avec mélange intime au moins une fois par jour, on fait fermenter en totalité à une température appropriée pour la levure employée,

c) ensuite la totalité de la masse obtenue après l'étape b) est distillée,

d) de l'alcool est ajouté au distillat,

e) l'ensemble du résidu de distillation est séché, brûlé et incinéré à une température appropriée, avant qu'il soit ajouté au distillat,

caractérisé en ce que

f) avant l'étape a) on détermine la masse sèche (M•T) de la plante utilisée et sur la base de celle-ci on prédéfinit la quantité de produit final (U), la quantité de produit intermédiaire (F1) d'une première fraction d'une distillation et une quantité d'hydrates de carbone à ajouter, ainsi que la quantité de levure,

g) on distille sous vide, de façon à recueillir deux fractions, dont la première contient tout l'alcool provenant de la fermentation et la seconde fraction consiste en la fraction du distillat obtenue après collecte de la première fraction,

h) on détermine la teneur en alcool de la première fraction et on y ajoute de l'alcool jusqu'à atteindre une teneur théorique en alcool par rapport à la quantité de produit final (U),

i) on incinère le résidu de distillation en évitant une scorification ou une fusion, et on extrait totalement des cendres, avec un solvant spécifique, l'ensemble des sels minéraux solubles qui s'y trouvent et on les purifie ensuite, après quoi on réunit à nouveau les extraits de sels purifiés et on les amène à sec,

k) on dissout les sels séchés dans une partie de la première fraction mélangée avec de l'alcool suffisante pour la dissolution et on sépare par filtration un précipité se formant éventuellement,

l) on mélange la solution avec le reste de la première fraction alcoolique et

m) pour finir on ajoute assez de la deuxième fraction pour que la masse totale corresponde à la quantité de produit final prédéfinie.

**2.** Procédé selon la revendication 1, caractérisé en ce qu'on emploie comme hydrates de carbone fermentescibles du saccharose au du miel.

**3.** Procédé selon la revendication 1, caractérisé en ce qu'on emploie code solvant spécifique de l'eau purifiée.

**4.** Procédé selon la revendication 3, caractérisé en ce que les extraits de sels réunis et amenés à sec sont à nouveau dissous sous ébullition dans de l'eau purifiée additionnelle, un précipité se formant éventuellement est séparé par filtration et la solution est amenée à sec.

**5.** Procédé selon la revendication 4, caractérisé en ce que les extraits de sels sont à nouveau dissous sous ébullition dans de l'eau purifiée, un précipité se formant éventuellement est séparé par filtration et la solution est amenée à sec, et ces processus sont répétés jusqu'à ce que la totalité des sels se dissolvent sans résidu lors d'une ébullition dans de l'eau purifiée et cette solution est ensuite amenée à sec.

**6.** Procédé selon l'une quelconque des revendications 3 à 5, caractérisé en ce que les extraits de sels réunis et amenés à sec sont à chaque fois calcinés avant dissolution dans de l'eau purifiée, sans être soumis à scorification ni à fusion.

**7.** Procédé selon l'une quelconque des revendications 3 à 6, caractérisé en ce que l'eau purifiée, employée pour éliminer les impuretés coextraites dans l'extraction des sels est remplacée par une partie de la seconde fraction.

8. Procédé selon la revendication 1, caractérisé en ce que

a) on emploie comme solvant, en excès, du vinaigre de vin ou un autre vinaigre, distillé et obtenu au moyen d'acétobacter à partir de liquides alcooliques,

b) on réunit les extraits de sels acides,

c) on amène à sec sous vide,

d) on dissout les sels séchés dans de l'eau purifiée pour éliminer les derniers restes d'acide acétique en excès et

e) on amène à nouveau à sec sous vide,

f) on répète les étapes d et e jusqu'à ce que le distillat donne une réaction pratiquement neutre.

9. Procédé selon la revendication 8, caractérisé en ce que les sels séchés et débarrassés de tout acide en excès sont dissous sous ébullition dans de l'eau purifiée pour en éliminer les impuretés coextraites, un précipité se formant éventuellement est séparé par filtration et la solution est amenée à sec.

10. Procédé selon la revendication 9, caractérisé en ce que les sels sont à nouveau dissous sous ébullition dans de l'eau purifiée, un précipité se formant éventuellement est séparé par filtration et la solution est amenée à sec, et ces processus sont répétés jusqu'à ce que la totalité des sels se dissolvent sans résidu lors d'une ébullition dans de l'eau purifiée et cette solution est ensuite amenée à sec.